(19) Europäisches Patentamt
European Patent Office
Office européen des brevets

(11) Numéro de publication : **0 282 390 B1**

(12) # FASCICULE DE BREVET EUROPEEN

(45) Date de publication du fascicule du brevet : **08.05.91 Bulletin 91/19**

(51) Int. Cl.$^5$ : **C07D 307/83,** C07D 405/12, C07D 407/12, A61K 31/365

(21) Numéro de dépôt : **88400441.7**

(22) Date de dépôt : **26.02.88**

(54) **Nouveaux dérivés de l'acide (dihydro-2,3 oxo-2 benzofurannyl-3)-2 acétique et les compositions pharmaceutiques qui les contiennent.**

(30) Priorité : **27.02.87 FR 8702630**

(43) Date de publication de la demande : **14.09.88 Bulletin 88/37**

(45) Mention de la délivrance du brevet : **08.05.91 Bulletin 91/19**

(84) Etats contractants désignés : **AT BE CH DE FR GB IT LI LU NL SE**

(56) Documents cités : **WO-A-80/01566**

(56) Documents cités : **CHEMICAL ABSTRACTS, vol. 93, no. 5, 4 août 1980, page 108, résumé no. 37433q, Columbus, Ohio, US; V. MACMILLAN: "Effects of gamma-hydroxybutyrate and gamma-butyrolactone on cerebral energy metabolism during exposure and recovery from hypoxemia-oligemia", & STROKE 1980, 11(3), 271-7**
**JOURNAL OF MEDICINAL CHEMISTRY, vol. 25, no. 5, mai 1982, pages 567-579, American Chemical Society, Washington, DC, US; E.J. CRAGOE, Jr. et al.: "Agents for the treatment of brain injury. 1. (Aryloxy)alkanoic acids"**

(73) Titulaire : **ADIR ET COMPAGNIE**
**22, rue Garnier**
**F-92201 Neuilly sur Seine (FR)**

(72) Inventeur : **Lavielle, Gilbert**
**1 Avenue Lilly**
**F-78170 La Celle Saint-Cloud (FR)**
Inventeur : **Lepagnol, Jean**
**5 Rue M. De Vlamick**
**F-78400 Chatou (FR)**

**EP 0 282 390 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux dérivés de l'acide (dihydro-2,3, oxo-2 benzofurannyl-3)-2 acétique, leurs procédés de préparation et les compositions pharmaceutiques qui les contiennent.

D'une façon surprenante, la littérature mentionne peu de dérivés du dihydro-2,3 oxo-2 benzofuranne pharmacologiquement actifs. Quelques dérivés de l'aminoalkyl-3 dihydro-2,3 oxo-2 benzofuranne doués d'une activité antispasmodique et anesthésique locale sont décrits dans le brevet US 2,513,698.

La demanderesse a maintenant découvert que certains dérivés de l'acide (dihydro-2,3 oxo-2 benzofurannyl-3)-2 acétique possèdent des propriétés pharmacologiques très intéressantes. En effet, les composés de la présente invention exercent des effets antihypoxiques et nootropes sans intervention des effets vasculaires. Ils s'opposent nettement à la mort cérébrale et à la défaillance énergétique tissulaire lors d'insuffisance d'apport en oxygène et trouvent leur application dans la correction des désordres liés à l'hypoxémie et à l'insuffisance énergétique, par exemple lors du vieillissement cérébral.

La présente invention a plus particulièrement pour objet les dérivés de l'acide (dihydro-2,3 oxo-2 benzofurannyl-3)-2 acétique, de formule générale I

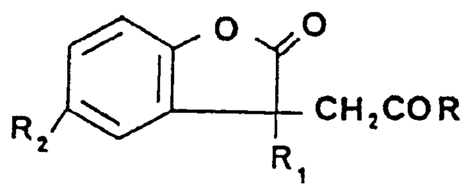

(I)

dans laquelle :

- $R_1$ représente un atome d'hydrogène, un radical alkyle linéaire au ramifié, de 1 à 4 atomes de carbone ou un radical phényle éventuellement substitué par un atome d'halogène, par un radical alcoxy renfermant de 1 à 4 atomes de carbone ou un radical alkyle de 1 à 4 atomes de carbone,
- $R_2$ représente un atome d'hydrogène ou d'halogène, un radical hydroxy, un radical alkyle de 1 à 4 atomes de carbone ou un radical alcoxy renfermant de 1 à 4 atomes de carbone,
- R représente

ou

un radical hydroxyle, un radical alcoxy, linéaire ou ramifié, de 1 à 4 atomes de carbone, ou un radical benzyloxy,

ou

un radical de formule générale A

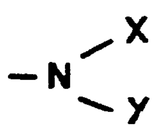

(A)

dans laquelle,

- X et Y identiques ou différents représentent chacun
  - un radical alkyle linéaire ou ramifié renfermant de 1 à 5 atomes de carbone,
  - un radical de formule générale $A_1$

$$-CHZ-COOH \qquad (A_1)$$

dans laquelle Z représente un atome d'hydrogène, un radical alkyle linéaire ou ramifié de 1 à 4 atomes

de carbone, un radical hydroxyalkyle de 1 à 4 atomes de carbone, un radical imidazolyle-4 méthylène, ou un radical benzyle éventuellement substitué par un radical alkyle de 1 à 4 atomes de carbone ou par un radical hydroxyle,

• un radical de formule générale $A_2$

$$-CH_2W \qquad (A_2)$$

dans laquelle W représente un radical dialkylaminométhylène linéaire ou ramifié de 3 à 9 atomes de carbone, un radical pyrrolidinyle-2 éventuellement substitué par un alkyle de 1 à 4 atomes de carbone, un radical benzyle, un radical isochromannyle-1 ou un radical isoquinolyle-1,

• ou forment ensemble avec l'azote auquel ils sont attachés un radical carboxy-2 pyrrolidinyle-2, un radical morpholinyle-4, un radical pipéridino éventuellement substitué par un radical phényle ou alcoxyphényle de 7 à 10 atomes de carbone, ou un radical pipérazinyle-1 (éventuellement substitué en 4 par un radical hydroxyalkyle de 1 à 4 atomes de carbone, un radical carboxyalkyle de 2 à 5 atomes de carbone, un radical benzyle éventuellement substitué par un radical alkyle de 1 à 4 atomes de carbone, un radical méthylène dioxy-3,4 benzyle, un radical phényle éventuellement substitué par un radical alkyle de 1 à 4 atomes de carbone ou par un radical trifluorométhyle ou par un atome d'halogène, ou un radical pyridyle-2 éventuellement substitué par un radical alkyle de 1 à 4 atomes de carbone ou par un radical trifluorométhyle),

sous forme racémique ou d'isomères optiques,

et leurs sels d'addition à un acide minéral ou organique, lorsqu'ils contiennent un groupement salifiable basique ou leurs sels d'addition à une base minérale ou organique, lorsqu'ils contiennent un groupement salifiable acide.

La présente invention a également pour objet le procédé de préparation des composés de formule générale I, caractérisé en ce que

l'on condense une benzofurannone-2 de formule générale II

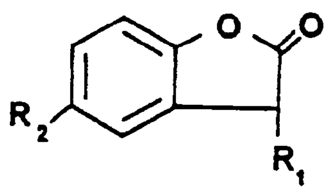

(II)

dans laquelle $R_1$ et $R_2$ ont la signification précédemment définie pour la formule I, avec un bromoacétate d'alkyle ou de benzyle de formule générale III,

$$BrCH_2COR' \qquad (III)$$

dans laquelle $R^1$ représente un radical alcoxy de 1 à 4 atomes de carbone ou un radial benzyloxy,

pour former un dérivé de formule générale Ia

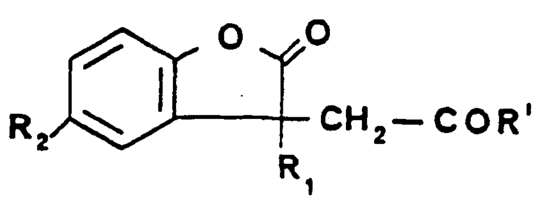

(Ia)

dans laquelle la définition des substituants R', $R_1$ et $R_2$ demeure celle mentionnée ci-dessus, lequel ensuite,

que l'on peut soumettre à une hydrogénation catalytique pour obtenir un dérivé de l'acide (dihydro-2,3 oxo-2 benzofurannyl-3)-2 acétique de formule générale Ib,

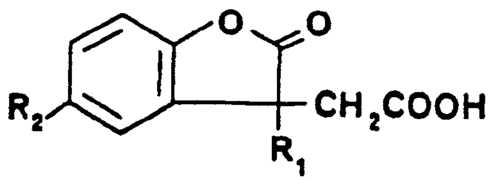

(Ib)

dans laquelle $R_1$, et $R_2$ ont la signification précédemment définie,

et ensuite, pour former les amides correspondants,

que l'on soumet d'abord à l'action du chlorure de thionyle pour obtenir un halogénure d'acyle de formule générale IV

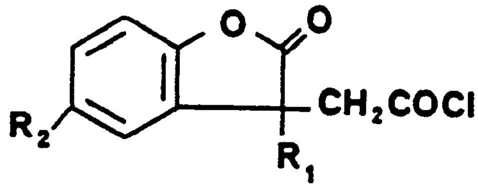

(IV)

dans laquelle la définition des substituants $R_1$, et $R_2$ demeure celle mentionnée ci-dessus et que l'on condense ensuite avec une amine secondaire de formule générale V

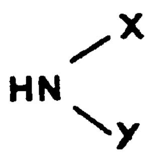

(V)

dans laquelle X et Y ont la signification précédemment définie pour la formule I pour former les composés de formule générale Ic

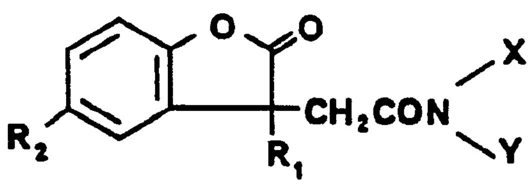

(Ic)

dans laquelle la signification des substituants $R_1$, $R_2$, X et Y reste identique à celle donnée précédemment.

L'ensemble des composés de formule Ia, Ib et Ic forme d'ensemble des composés de formule I,

qui quand ils contiennent une fonction acide ou basique, peuvent être salifiés respectivement par une base ou un acide minéral ou organique, pharmaceutiquement acceptable,

ou séparés en leurs isomères optiques puis éventuellement salifiés.

Les benzofurannones-2 de formule générale II, lorsqu'elles ne sont pas disponibles dans le commerce, peuvent être préparées selon des procédés connus. (J. Med. Chem.,(1972),5, p 551 ; Brevet US 2,513,698 du

04.07:1950 ; Ber. (1897), 30, p 124).

La condensation des composés de formule générale II avec les bromoacétates d'alkyle ou de benzyle s'effectue en présence d'un hydrure métallique, tel que l'hydrure de sodium, dans un solvant organique anhydre et à la température ambiante, selon des procédés connus. (J. Chem. Soc. (1973),p 711 ; J. Org. Chem. (1961), 26, p 4821).

L'hydrogénolyse des composés de formule générale Ia s'effectue en présence de palladium à 10% sur charbon, à température ambiante et sous pression atmosphérique.

La condensation des chlorures d'acides de formule générale IV avec les amines de formule générale V s'effectue ou dans un solvant organique anhydre tel que le benzène, ou en milieu aqueux alcalin quand les composés de formule générale V sont des amino-acides.

Parmi les acides pharmaceutiquement acceptables pour la préparation des sels d'addition aux composés de formule générale I, on peut citer les acides chlorhydrique, phosphorique, citrique, oxalique, sulfurique, tartrique, maléïque, mandélique, méthane-sulfonique, etc...

Parmi les bases pharmaceutiquement acceptables pour la préparation des sels des composés de formule générale I, on peut citer les hydroxydes de sodium, potassium, ammonium etc...

Les composés selon l'invention, ainsi que leurs sels sont doués de propriétés pharmacologiques fort intéressantes et se distinguent des autres dérivés de dihydro-2,3 oxo-2 benzofuranne déjà connus. En effet, les essais pharmacologiques in vivo ont montré que les composés de la présente invention exercent un puissant effet antihypoxique chez l'animal.

Lors du vieillissement ou à la suite d'un accident vasculaire cérébral, la fragilité et la vulnérabilité cellulaire accrues sont des composantes physiopathologiques importantes pour la recherche de nouvelles thérapeutiques visant à protéger le cerveau mis alors en situation d'incapacité à répondre à toute nouvelle agression issue de son environnement.

Une telle agression peut être reproduite sous forme d'un défaut d'apport en oxygène et c'est pourquoi, par leurs conséquences, une analogie étroite existe entre l'hypoxie etle vieillissement cérébral. Cette analogie s'exprime notamment par la baisse des réserves énergétiques du cerveau, la moindre résistance au stress et la baisse du renouvellement de la synthèse oxygéno-dépendante des neurotransmetteurs.

Les composés de la présente invention ont été testés sur leur capacité à prolonger la survie du tissu cérébral lors d'hypoxie aiguë chez la souris, ou à maintenir le taux de composés énergétiques tissulaires chez le rat soumis à une baisse d'apport en oxygène (Pharmacologie of Cerebral Ischemia, p. 334-339, (1968) Elservier Science Publishers B.V., J. Krieglstein edt). Dans les deux types d'expérience réalisée, les composés de l'invention ont été comparés à des composés de référence qui sont le méclofénoxate, le pyritinol et le piracétam (Arz. Forsch. Drug.Res. (1986), 36 II, N° 9, p. 1314-1320).

Ces derniers ont été choisis pour leurs indications thérapeutiques vis-à-vis des symptômes associés à la sénescence ou aux séquelles d'accident vasculaire cérébral, indications revendiquées sur la base de l'effet antihypoxique et nootrope sans intervention d'effet vasculaire. Les composés de type myolytique ou adrénolytique ont donc été écartés.

Les essais pharmacologiques chez la souris ont prouvé que les composés de la présente invention ont un effet protecteur antihypoxique 2 à 8 fois plus puissant par rapport à celui du composé de référence le plus actif. Chez le rat hypoxié, les composés de l'invention ont exercé les mêmes effets protecteurs sur l'énergie cérébrale que les composés de référence, mais à des doses qui sont 3 à 10 fois inférieures et ont ainsi confirmé le grand intérêt de leur emploi en thérapeutique.

En s'opposant nettement à la mort cérébrale et à la défaillance énergétique tissulaire lors d'insuffisance d'apport en oxygène, les composés de la présente invention exercent un effet antihypoxique marqué et sont donc utiles dans les cas de syndromes ischémiques de toute localisation, aiguë, transitoire ou progressive, puisqu'ils exercent leurs propriétés pharmacologiques vis-à-vis de la défaillance d'oxygénation qui accompagne ces accidents. Leurs propriétés pharmacologiques permettent leur application dans la correction des désordres liés à l'hypoxémie et à l'insuffisance énergétique par exemple lors du vieillissement cérébral.

L'invention s'étend aussi aux compositions pharmaceutiques renfermant comme principe actif au moins un composé de formule générale I ou l'un de ses sels avec une base ou acide minéral ou organique pharmaceutiquement compatible, en association avec un ou plusieurs excipients inertes, et appropriés.

Les compositions pharmaceutiques ainsi obtenues sont présentées avantageusement sous des formes diverses telles que par exemple comprimés, dragées, gélules, glossettes ou autres préparations galéniques appropriées pour une administration sublinguale, suppositoires, solutions injectables ou buvables.

La posologie peut varier largement en fonction de l'âge, du poids du patient, de la nature et de la sévérité de l'affection ainsi que de la voie d'administration.

La voie d'administration préférée est la voie orale ou parentérale. D'une manière générale, la posologie unitaire s'échelonnera entre 0,5 et 300 mg et la posologie journalière, utilisable en thérapeutique humaine entre

0,5 et 900 mg.

Les exemples suivants, donnés à titre non-limitatif, illustrent l'invention.

Les points de fusion indiqués sont mesurés selon la technique Micro-Köfler. Les spectres infrarouge sont obtenus avec des solutions des produits dans le Nujol. Les spectres de résonance magnétique nucléaire du proton (R.M.N) ont été enregistrés à 60 MHz. Les constantes physiques spectrales des composés de formule générale 1 sont indiquées dans le Tableau 1.

**EXEMPLE 1 :**

Acide (chloro-5-dihydro-2,3 oxo-2 phényl-3 benzofurannyl-3)-2 acétique

Stade A :

Ester benzylique de l'acide (chloro-5 dihydro-2,3 oxo-2 phényl-3 benzofurannyl-3)-2 acétique

A une suspension de 0,204 mole d'hydrure de sodium dans 100 ml de diméthylformamide anhydre, on ajoute une solution de 0,204 mole de chloro-5 dihydro-2,3 oxo-2 phényl-3 benzofuranne dans 200 ml de diméthylformamide anhydre. On maintient le milieu sous agitation à la température ambiante pendant une heure, puis on ajoute lentement 0,224 mole de bromoacétate de benzyle et on abandonne le milieu 12 heures à température ambiante. On concentre le mélange réactionnel sous vide, on hydrolyse à l'aide d'un litre d'eau, on extrait la phase aqueuse avec deux fois 250 ml de benzène, on sèche la phase organique sur sulfate de sodium anhydre et on évapore le solvant. Le résidu est lavé à l'éther isopropylique et ensuite filtré. Ainsi sont obtenus 0,173 mole.

| | |
|---|---|
| Rendement : | 85% |
| Point de fusion : | 95°C |

Stade B :

Dans un ballon de 2 litres on mélange 0,127 mole de l'ester benzylique obtenu au stade précédent, 2 g de palladium sur charbon à 10% et 700 ml d'éthanol anhydre. On hydrogène le milieu à température ambiante sous pression atmosphérique. La quantité nécessaire d'hydrogène absorbée, on filtre le catalyseur et on évapore le solvant. On lave le résidu cristallin à l'éther de pétrole et on recueille après filtration 35 g de cristaux.

| | |
|---|---|
| Rendement : | 90% |
| Point de fusion : | 199°C |

**EXEMPLES 2 à 6 :**

Les composés suivants ont été préparés selon le procédé décrit dans l'exemple 1, en utilisant au stade A les dérivés de dihydro-2,3 oxo-2 phényl-3 benzofuranne adéquats.

**EXEMPLE 2 :**

Acide (dihydro-2,3 fluoro-5 oxo-2 phényl-3 benzofurannyl-3)-2 acétique

| | |
|---|---|
| Rendement : | 85% |
| Point de fusion : | 228°C |

**EXEMPLE 3 :**

Acide (dihydro-2,3 oxo-2 phényl-3 benzofurannyl-3)-2 acétique

| | |
|---|---|
| Rendement : | 95% |
| Point de fusion : | 184°C |

**EXEMPLE 4 :**

Acide [(chloro-4 phényl)-3 dihydro-2,3 oxo-2 benzofurannyl-3]-2 acétique

| | |
|---|---|
| Rendement : | 90% |
| Point de fusion : | 192°C |

**EXEMPLE 5 :**

Acide (dihydro-2,3 hydroxy-5 oxo-2 phényl-3 benzofurannyl-3)-2 acétique

| | |
|---|---|
| Rendement : | 90% |
| Point de fusion : | 226°C |

**EXEMPLE 6 :**

Acide (dihydro-2,3 méthoxy-5 oxo-2 phényl-3 benzofurannyl-3)-2 acétique

| | |
|---|---|
| Rendement : | 85% |
| Point de fusion : | 188°C |

**EXEMPLE 7 :**

Ester benzylique de l'acide (dihydro-2,3 hydroxy-5 oxo-3 phényl-3-benzofurannyl-3)-2 acétique

Ce composé a été préparé à partir de la dihydro-2,3 hydroxy-5 oxo-2 phényl-3benzofuranne selon le procédé décrit au stade A dans l'exemple 1.

| | |
|---|---|
| Rendement : | 33% |
| Point de fusion : | 96°C |

**EXEMPLE 8 :**

Ester éthylique de l'acide (chloro-5 dihydro-2,3 oxo-2 phényl-3 benzofurannyl-3)-2 acétique

A une suspension de 42,8 mmoles d'hydrure de sodium dans 20 ml de diméthylformamide anhydre, on ajoute à température ambiante une solution de 42,8 mmoles de chloro-5 dihydro-2,3 oxo-2 phényl-3 benzofuranne dans 50 ml des diméthylformamide anhydre. On agite une heure à 20°C et on ajoute 42,8 mmoles de bromoacétate d'éthyle. On agite 12 heures à 20°C, on concentre le milieu sous vide, on reprend le résidu dans 200 ml d'une solution chlorhydrique à 1%, on extrait la phase aqueuse au benzène, on sèche la phase organique sur sulfate de sodium anhydre, on évapore le solvant, on lave le résidu cristallin avec une solution d'éther éthylique et d'acétone (90/10). Après filtration, on recueille 25,8 mmoles de cristaux.

| | |
|---|---|
| Rendement : | 60% |
| Point de fusion : | 130°C |

**EXEMPLE 9 :**

(chloro-5 dihydro-2,3 oxo-2 phényl-3 benzofurannyl-3)-2 [(trifluorométhyl-5 pyridinyl-2)-4 pipérazinyl]-1 oxo-1 éthane

STADE A

Chlorure de (chloro-5 dihydro-2,3 oxo-2 phényl-3 benzofurannyl-3)-2 acétyle

A une solution agitée et chauffée à 70°C de 30,2 g d'acide (chloro-5 dihydro-2,3 oxo-2 phényl-3 benzofurannyl-3)-2 acétique dans 250 ml de benzène anhydre, on ajoute goutte à goutte 23,7 g chlorure de thionyle bidistillé. L'addition terminée, on chauffe au reflux jusqu'à complète disparition du dégagement gazeux. On concentre le milieu sous vide on lave le résidu avec 200 ml de benzène anhydre, on évapore le solvant. On répète deux fois cette opération et on laisse le produit cristalliser à température ambiante. On lave les cristaux avec de l'éther de pétrole, on filtre et on obtient 31 g de produit pur.

| | |
|---|---|
| Rendement : | 95% |
| Point de fusion : | 107°C |

STADE B

A une solution préalablement refroidie à 6°C de 10 g du chlorure d'acide obtenu au stade précédent dans 200 ml de benzène anhydre, on ajoute une solution de 7,5 g de (trifluorométhyl-3 pyridinyl-2)-4-pipérazine et de 3,15 g de triéthylamine dans 100 ml de benzène anhydre. On agite 2 heures à 20°C, on concentre sous vide, on neutralise le résidu à l'aide d'une solution d'hydroxyde de sodium 1N, on extrait la phase aqueuse deux fois à l'aide de 200 ml de dichlorométhane, on lave la phase organique à l'eau, on la sèche sur sulfate de sodium anhydre et on évapore le solvant. On recristallise le produit brut dans l'éthanol pour obtenir 5 g de produit.

| | |
|---|---|
| Rendement : | 30% |
| Point de fusion : | 228°C |

**EXEMPLE 10**

N,N diéthyl (chloro-5 dihydro-2,3 oxo-2 phényl-3 benzofurannyl-3)-2 acétamide

Ce composé a été préparé selon le procédé décrit dans l'exemple 9, en utilisant au Stade B la diéthylamine.

| | |
|---|---|
| Rendement : | 50% |
| Point de fusion : | 145°C |

**EXEMPLE 11**

N-méthyl, N-[(chloro-5 dihydro-2,3 oxo-2 phényl-3 benzofurannyl-3)-2 acétyl] leucine

A une solution de 3 g de N-méthyl leucine dans 70 ml d'eau et 40 ml de tétrahydrofuranne on ajoute 0,9 g d'hydroxyde de sodium. Le milieu est ensuite refroidi à 5°C et on ajoute alors simultanément et par petites quantités une solution de 7,3 g de chlorure de (chloro-5 dihydro-2,3 oxo-2 phényl-3 benzofurannyl-3)-2 acétyle dans 50 ml de tétrahydrofuranne et une solution de 0,9 g d'hydroxyde de sodium dans 22,7 ml d'eau de façon à maintenir le pH à 12. On agite 3 heures à température ambiante et on ajoute 22,7 ml d'acide chlorhydrique N. On concentre le milieu, on filtre le précipité obtenu, on le dissout à chaud dans un mélange éthanol-méthanol (50/50). On filtre et on évapore le filtrat. On obtient le produit cristallin.

| | |
|---|---|
| Rendement : | 65% |
| Point de fusion : | 183°C |

**EXEMPLE 12**

Chlorhydrate du N-méthyl N-diéthylaminoéthyl (chloro-5 dihydro-2,3 oxo-2 phényl-3 benzofurannyl-3)-2 acétamide

Ce composé a été préparé selon le procédé décrit dans l'exemple 9, en utilisant au Stade B, la N-méthyl N-diéthylaminoéthyl amine et ensuite en salifiant le produit obtenu avec l'acide chlorhydrique en solution dans l'éthanol.

| | |
|---|---|
| Rendement : | 70% |
| Point de fusion : | 190°C |

**EXEMPLE 13**

N-[(chloro-5 dihydro-2,3 oxo-2 phényl-3 benzofurannyl-3)-2 acétyl] proline

Ce composé a été préparé selon le procédé décrit dans l'exemple 11, en utilisant comme aminoacide la proline.

Rendement : 65%
Point de fusion : > 260°C

**EXEMPLES 14-19**

Les composés suivants ont été préparés selon le procédé décrit dans l'exemple 9, en utilisant au Stade B, les amines adéquates.

**EXEMPLE 14**

(chloro-5 dihydro-2,3 oxo-2 phényl-3 benzofurannyl-3)-2 morpholino-1 oxo-1 éthane

Rendement : 76%
Point de fusion : 211°C

**EXEMPLE 15**

Chlorhydrate du (chloro-5 dihydro-2,3 oxo-2 phényl-3 benzofurannyl-3)-2 [(hydroxy-2 éthyl)-4 pipérazinyl]-1 oxo-1 éthane

Rendement : 25%
Point de fusion : 235°C

**EXEMPLE 16**

Chlorhydrate du (chloro-5 dihydro-2,3 oxo-2 phényl-3 benzofurannyl-3)-2 [(méthylène dioxy-3,4 benzyl)-4 pipérazinyl]-1 oxo-1 éthane

Rendement : 55%
Point de fusion : > 260°C

**EXEMPLE 17**

(chloro-5 dihydro-2,3 oxo-2 phényl-3 benzofurannyl-3)-2 (benzyl-4 pipérazinyl)-1 oxo-1 éthane

Rendement : 65%
Point de fusion : 155°C

**EXEMPLE 18**

Chlorhydrate du (chloro-5 dihydro-2,3 oxo-2 phényl-3 benzofurannyl-3)-2 [(trifluorométhyl-3 phényl)-4 pipérazinyl]-1 oxo-1 éthane

Rendement : 90%
Point de fusion : 192°C

**EXEMPLE 19**

Chlorhydrate du (chloro-5 dihydro-2,3 oxo-2 phényl-3 benzofurannyl-3)-2 [(méthyl-3 phényl)-4 pipérazinyl]-1 oxo-1 éthane

| | |
|---|---|
| Rendement : | 40% |
| Point de fusion : | 261°C |

**EXEMPLE 20**

Chlorhydrate du (fluoro-5 dihydro-2,3 oxo-2 phényl-3 benzofurannyl-3)-2 [(méthylène dioxy-3,4 benzyl)-4-pipérazinyl]-1 oxo-1 éthane

Ce composé a été préparé selon le procédé décrit dans l'exemple 9 à partir du chlorure de (fluoro-5 dihydro-2,3 oxo-2 phényl-3 benzofurannyl-3)-2 acétyle et la (méthylène dioxy-3,4 benzyl)-4 pipérazine.

| | |
|---|---|
| Rendement : | 45% |
| Point de fusion : | 195°C |

**EXEMPLE 21**

N-méthyl N-[(dihydro-2,3 oxo-2 phényl-3 benzofurannyl-3)-2 acétyl] glycine

Ce composé a été préparé selon le procédé décrit dans l'exemple 11 à partir du chlorure de (dihydro-2,3 oxo-2 phényl-3 benzofurannyl-3)-2 acétyle et la N-méthyl glycine.

| | |
|---|---|
| Rendement : | 55% |
| Point de fusion : | 120°C |

**EXEMPLE 22**

Chlorhydrate du (dihydro-2,3 oxo-2 phényl-3 benzofurannyl-3)-2 [(méthylène dioxy-3,4 benzyl)-4 pipérazinyl]-1 oxo-1 éthane

Ce composé a été préparé à partir du chlorure de (dihydro-2,3 oxo-2 phényl-3 benzofurannyl-3)-2 acétyle et la (méthylène dioxy-3,4 benzyl)-4 pipérazine selon le procédé décrit dans l'exemple 9.

| | |
|---|---|
| Rendement : | 45% |
| Point de fusion : | 192°C |

**EXEMPLE 23**

Chlorhydrate du (dihydro-2,3 oxo-2 benzofurannyl-3)-2 [(méthylène dioxy-3,4 benzyl)-4 pipérazinyl]-1 oxo-1 éthane

STADE A

Ester benzylique de l'acide (dihydro-2,3 oxo-2 benzofurannyl-3)-2 acétique

On refroidit sous courant d'azote un mélange de 7,53 g de diisopropylamine dans 150 ml de tétrahydrofuranne anhydre à–60°C. On ajoute goutte à goutte à cette température 46,6 ml d'une solution 1,6 M de n-butyllithium dans l'hexane. L'addition terminée, on laisse remonter la température du milieu réactionnel à 5°C, on agite le mélange 10 minutes à cette température, on le refroidit à –80°C et on ajoute goutte à goutte une solution de 10 g de dihydro-2,3 benzofurannone-2 dans 150 ml de tétrahydrofuranne anhydre. On maintient 10 minutes à –80°C puis 10 minutes à –60°C. On refroidit à nouveau à –80°C et on ajoute une solution de 18,8 g de bromoacétate de benzyle dans 16,05 g d'hexaméthylphosphorotriamide. On laisse remonter la température à 20°C, on agite 1 heure à cette température et on hydrolyse le mélange réactionnel à 0°C à l'acide de 26 ml

d'une solution saturée de chlorure d'ammonium. Le milieu est ensuite versé sur 2,5 l d'une solution d'acide chlorhydrique à 1%. On filtre le précipité, on le dissout dans 200 ml de benzène, on lave la phase organique à l'eau, on la sèche sur sulfate de sodium anhydre et on évapore le solvant. Le résidu est recristallisé dans l'éther de pétrole.

| | |
|---|---|
| Rendement : | 90% |
| Point de fusion : | 88°C |

STADE B

Acide (dihydro-2,3 oxo-2 benzofurannyl)-3)-2 acétique

Cet acide est obtenu à partir du composé synthétisé au stade précédent, selon le procédé décrit dans l'exemple 1, Stade B.

STADE C

Chlorure de (dihydro-2,3 oxo-2 benzofurannyl-3)-2 acétyle

Ce composé a été préparé selon le procédé décrit dans l'exemple 9, Stade A et à partir de l'acide (dihydro-2,3 oxo-2 benzofurannyl-3)-2 acétique.

STADE D

A une solution préalablement refroidie à 10°C de 4,5 g de chlorure de (dihydro-2,3 oxo-2 benzofurannyl-3)-2 acétyle dans 50 ml de benzène anhydre, on ajoute une solution de 9,4 g de (méthylène dioxy-3,4 benzyl)-4 pipérazine dans 100 ml de benzène anhydre. On agite 12 heures à température ambiante, on filtre le précipité de chlorhydrate d'amine, on le lave au benzène plusieurs fois. Les phases organiques rassemblées sont lavées à l'eau, séchées sur sulfate de sodium anhydre et concentrées sous vide. Le produit brut obtenu est chromatographié sur 300 g de silice 70-230 mesh (éluant : dichlorméthane 9,5 V méthanol 2,5 V). On dissout le produit résultant (3 g) dans le minimum d'acétone et on le cristalline sous forme de chlorhydrate par addition de 1,5 ml d'éthanol chlorhydrique 5,07 N.

| | |
|---|---|
| Rendement : | 35% |
| Point de fusion : | 188°C |

**EXEMPLES 24-25**

Ces composés ont été préparés par condensation du chlorure de (dihydro-2,3 oxo-2 benzofurannyl-3)-2 acétyle dans les amines adéquates selon le procédé décrit dans l'exemple 23, Stade D.

**EXEMPLE 24**

(dihydro-2,3 oxo-2 benzofurannyl-3)-2 morpholino-1 oxo-1 éthane

| | |
|---|---|
| Rendement : | 50% |
| Point de fusion : | 103°C |

**EXEMPLE 25**

Chlorhydrate du (dihydro-2,3 oxo-2 benzofurannyl-3)-2 [(méthyl-3 benzyl)-4 pipérazinyl]-1 oxo-1 éthane

| | |
|---|---|
| Rendement : | 50% |
| Point de fusion : | 138°C |

**EXEMPLE 26**

Chlorhydrate du (chloro-5 dihydro-2,3 oxo-2 benzofurannyl-3)-2 [(méthyl-3 phényl)-4 pipérazinyl]-1 oxo-1 éthane

Ce composé a été préparé selon le procédé décrit dans l'exemple 23 en utilisant au Stade A la chloro-5 dihydro-2,3 benzofurannone-2 et au Stade D la (méthyl-3 phényl)-4 pipérazine.

| | |
|---|---|
| Rendement : | 25% |
| Point de fusion : | 160°C |

**EXEMPLE 27**

[(chloro-5 dihydro-2,3 oxo-2 phényl-3 benzofurannyl-3)-2 [(trifluorométhyl-4 pyridinyl-2)-4 pipérazinyl]-1 oxo-1 éthane

Ce composé a été préparé par condensation du chlorure de (chloro-5 dihydro-2,3 oxo-2 phényl-3 benzofurannyl-3)-2 acétyle avec la (trifluorométhyl-4 pyridinyl-2)-4 pipérazine selon le procédé décrit dans l'exemple 9, Stade B.

| | |
|---|---|
| Rendement : | 40% |
| Point de fusion : | 202°C |

**EXEMPLE 28**

N-[(chloro-5 dihydro-2,3 oxo-2 phényl-3 benzofurannyl-3)-2 acétyl], N-méthyl phénylalanine

Ce composé a été préparé selon le procédé décrit dans l'exemple 11 en utilisant comme acide aminé la N-méthyl phénylalanine.

| | |
|---|---|
| Rendement : | 65% |
| Point de fusion : | 130-135°C |

**EXEMPLE 29 :**

Chlorhydrate du (chloro-5 dihydro-2,3 oxo-2 phényl-3 benzofurannyl-3)-2 (carboxyméthyl-4 pipérazinyl)-1 oxo-1 éthane

Ce composé a été préparé selon le procédé décrit dans l'exemple 11 en utilisant comme acide aminé l'acide pipérazinyl-2 acétique.

| | |
|---|---|
| Rendement : | 80% |
| Point de fusion : | 224°C |

**EXEMPLES 30-32**

Les composés indiqués ci-dessus ont été préparés par condensation du chlorure de (chloro-5 dihydro-2,3 oxo-2 phényl-3 benzofurannyl-3)-2 acétyle avec des amines adéquates selon le procédé décrit dans l'exemple 9, Stade B.

**EXEMPLE 30**

(chloro-5 dihydro-2,3 oxo-2 phényl-3 benzofurannyl-3)-2 [(méthoxy-3 phényl)-3 pipéridinyl]-1 oxo-1 éthane

| | |
|---|---|
| Rendement : | 80% |
| Point de fusion : | 202°C |

**EXEMPLE 31**

(chloro-5 dihydro-2,3 oxo-2 phényl-3 benzofurannyl-3)-2 [(N-isochormannyl-1 méthyl) N-méthyl] amino-1 oxo-1 éthane

Rendement : 70%
Point de fusion : 159°C

**EXEMPLE 32**

Chlorhydrate du (chloro-5 oxo-2 phényl-3 benzofurannyl-3)-2 [(éthyl-1 pyrrolidinyl-2) méthyl] méthylamino-1 oxo-1 éthane

Rendement : 40%
Point de fusion : 218°C

**EXEMPLE 33**

(dihydro-2,3 méthyl-5 oxo-2 phényl-3 benzofurannyl-3)-2 [(fluoro-4 phényl)-4 pipérazinyl]-1 oxo-1 éthane

Ce composé a été préparé en condensant le chlorure de (dihydro-2,3 méthyl-5 oxo-2 phényl-3 benzofurannyl-3)-2 acétyle avec la (fluoro-4 phényl)-4 pipérazine selon le procédé décrit dans l'exemple 9, Stade B.

Rendement : 82%
Point de fusion : 120°C

**EXEMPLE 34**

Chlorhydrate du (dihydro-2,3 oxo-2 propyl-3 benzofurannyl-3)-2 [(méthylènedioxy-3,4 benzyl)-4 pipérazinyl]-1 oxo-1 éthane

STADE A

Ester de benzyle de l'acide (dihydro-2,3 oxo-2 propyl-3 benzofurannyl-3) acétique

A une suspension de 0,204 mole d'hydrure de sodium dans 100 ml de diméthylformamide anhydre, on ajoute une solution de 0,204 mole de dihydro-2,3 oxo-2 propyl-3 benzofuranne dans 200 ml de diméthylformamide anhydre. On maintient le milieu sous agitation à température ambiante pendant une heure puis on ajoute 0,224 mole du bromoacétate de benzyle et on abandonne le milieu 12 heures à température ambiante. On concentre le mélange réactionnel sous vide, on hydrolyse à l'acide d'un litre d'eau ; on extrait la phase aqueuse avec deux fois 250 ml de benzène, on sèche la phase organique sur sulfate de sodium anhydre et on évapore le solvant. Le résidu cristallisé lavé à l'éther isopropylique et filtré fournit 0,173 mole une huile jaune pâle.

Rendement : 85%

STADE B

Acide (dihydro-2,3 oxo-2 propyl-3 benzofurannyl-3) acétique

Dans un ballon de 2 l on mélange 50 g de l'ester benzylique obtenu au stade précédent, 2 g de palladium sur charbon à 10% et 700 ml d'éthanol anhydre. On hydrogène le milieu à température ambiante sous pression atmosphérique. La quantité nécessaire d'hydrogénure absorbé, on filtre le catalyseur et on évapore le solvant. On lave le résidu cristallin à l'éther de pétrole et on recueille après filtration les cristaux.

Rendement : 70%

Point de fusion : 117°C

STADE C

Chlorure de (dihydro-2,3 oxo-2 propyl-3 benzofurannyl-3)-2 acétyle

A une solution agitée et chauffée à 70°C de 23,4 g d'acide (dihydro-2,3 oxo-2 propyl-3 benzofurannyl-3) acétique dans 250 ml de benzène anhydre, on ajoute goutte à goutte 23,7 g de chlorure de thionyle bidistillé. L'addition terminée, on chauffe jusqu'à complète disparition du dégagement gazeux. On concentre le milieu sous vide, on lave le résidu avec 200 ml de benzène anhydre, on évapore le solvant. On répète deux fois cette opération, et on obtient une huile très épaisse qui ne cristalline pas.

Rendement : 95%

STADE D

A une solution préalablement refroidie à 6°C de 7,85 g (31,1 mmoles) du chlorure d'acide précédent dans 200 ml de benzène anhydre, on ajoute une solution de 6,85 g de (méthylènedioxy-3,4 benzyl)-4 pipérazine et de 3,15 g de triéthylamine dans 100 ml de benzène anhydre. On agite 2 h à 20°C, on concentre sous vide, on reprend au benzène, on lave à l'eau la phase organique, on sèche sur sulfate de sodium anhydre, et on évapore le solvant. On recristallise le résidu dans un mélange d'éther isopropylique et d'alcool isopropylique (47/10). On dissout les cristaux obtenus dans l'éthanol et on ajoute une quantité adéquate d'une solution d'acide chlorhydrique dans l'éthanol, on concentre le milieu réactionnel et on ajoute de l'éther éthylique pour cristalliser le chlorhydrate formé.

Rendement : 60%
Point de fusion : 170°-175°C

**EXEMPLES 35-37**

Ces composés ont été préparés par condensation du chlorure de [chloro-5 dihydro-2,3 oxo-2 (méthyl-4 phényl)-3 benzofurannyl-3]-2 acétyle avec les amines adéquates.

**EXEMPLE 35**

Chlorhydrate de la N-méthyl N-diéthylaminoéthyl [chloro-5 dihydro-2,3 oxo-2 (méthyl-4 phényl)-3 benzofurannyl-3]-2 acétamide

Rendement : 37%
Point de fusion : 140°C

**EXEMPLE 36**

Chlorhydrate de la N-méthyl N-diisopropylaminoéthyl [chloro-5 dihydro-2,3 oxo-2 (méthyl-4 phényl)-3 benzofurannyl-3] acétamide.

Rendement : 30%
Point de fusion : 190°C

**EXEMPLE 37**

Chlorhydrate du [chloro-5 dihydro-2,3 oxo-2 (méthyl-4 phényl)-3 benzofurannyl]-3-2 (hydroxy-2 éthyl)-4 pipérazinyl)-1 oxo-1 éthane.

Rendement : 33%

Point de fusion : > 260°C

**TABLEAU I**

| Ex | $R_1$ | R | $R_2$ | IR ($cm^{-1}$) ν C = 0 lactone | IR ($cm^{-1}$) ν C = 0 extracyclique | RMN (solvant) |
|---|---|---|---|---|---|---|
| 1 | 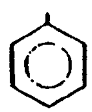 | - OH | Cl | 1800 | 1705 | ($CDCl_3$ + DMSO-$d_6$)<br>9,2 ppm,m,1H echangeable;<br>6,5 à 7,6 ppm,m,8H;<br>3,4 ppm,AB,2H; |
| 2 | 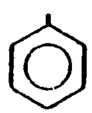 | - OH | F | 1810 | 1715 | ($CDCl_3$ + DMSO-$d_6$)<br>9,75 ppm,m,1H échangeable;<br>6,75 à 7,75 ppm,m,8H;<br>3,45 ppm,AB,2H |
| 3 | 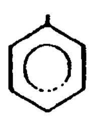 | - OH | H | 1805 | 1715 | ($CDCl_3$ + DMSO-$d_6$)<br>10,3 ppm,m,1H échangeable;<br>6,7 à 7,6 ppm,m,9H;<br>3,4 ppm,AB,2H |

**TABLEAU I**
**(Suite 1)**

| Ex | $R_1$ | R | $R_2$ | IR ($cm^{-1}$) | | RMN (solvant) |
|---|---|---|---|---|---|---|
| | | | | ν C = 0 lactone | ν C = 0 extracyclique | |
| 4 | 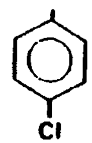 | - OH | - H | 1800 | 1710 | (DMSO-$d_6$)<br>12,5 ppm,m,1H échangeable;<br>6,9 à 7,7 ppm,m,8H; 3,6 ppm,s,2H |
| 5 | 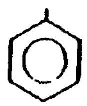 | - OH | - OH | 1785 | 1700 | ($CDCl_3$ + DMSO-$d_6$)<br>9,6 ppm,m,2H échangeables;<br>7,3 ppm,s,5H; 6,6 à 7,2 ppm,m,3H,<br>3,4 ppm,AB,2H |
| 6 | 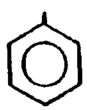 | - OH | - $OCH_3$ | 1800 | 1710 | ($CDCl_3$ + DMSO-$d_6$)<br>10,3 ppm,m,1H échangeable;<br>7,3 ppm,s,5H; 6,6 à 7,2 ppm,m,3H;<br>3,8 ppm,s,3H; 3,4 ppm,AB,2H |
| 7 | 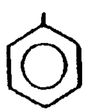 | - $OCH_2$ 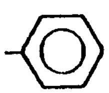 | - OH | 1760 | 1745 | ($CDCl_3$)<br>6,2 à 7,7 ppm,m,12H + 1H échangeable;<br>4,9 ppm,s,2H;<br>3,4 ppm,AB,2H |

**TABLEAU I**
**(Suite 2)**

| Ex | $R_1$ | R | $R_2$ | IR ($cm^{-1}$) $\nu C=0$ lactone | IR ($cm^{-1}$) $\nu C=0$ extracyclique | RMN (solvant) |
|---|---|---|---|---|---|---|
| 8 | 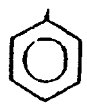 | $-O-C_2H_5$ | Cl | 1770 | 1730 | ($CDCl_3$)<br>6,6 à 7,8 ppm,m,8H; 4 ppm,q,2H;<br>3,5 ppm,AB,2H; 1,1 ppm,t,3H |
| 9 | 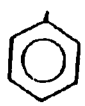 | 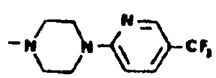 | Cl | 1805 | 1640 | (DMSO-$d_6$)<br>8,3 ppm,d,1H; 6,6 à 7,8 ppm,m,10H,<br>3,7 ppm,AB,2H; 3,6 ppm,m,4H,<br>3,4 ppm,m,4H |
| 10 | 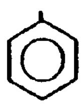 | 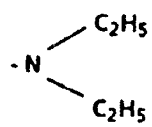 | Cl | 1805 | 1635 | ($CDCl_3$)<br>6,8 à 7,5 ppm,m,8H; 2,8 à 3,8 ppm,m,<br>6H; 1,2 ppm,t,3H; 1,1 ppm,t,3H |
| 11 | 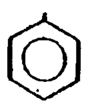 | 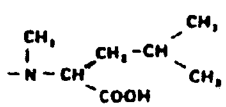 | Cl | 1815 | 1640 (amide)<br>1710 (acide) | ($CDCl_3$ + DMSO-$d_6$)<br>6,7 à 7,8 ppm,m,8H + 1H échangeable<br>4,8 ppm,m,1H; 3,7 ppm,m,2H;<br>2,9 ppm,s,3H; 1,5 ppm,m,3H;<br>0,9 ppm,m,6H |

**TABLEAU I**

**(Suite 3)**

| Ex | $R_1$ | R | $R_2$ | IR ($cm^{-1}$) | | RMN (solvant) |
|---|---|---|---|---|---|---|
| | | | | ν C = 0 lactone | ν C = 0 extracyclique | |
| 12 | 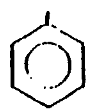 | 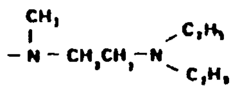 | - Cl | 1805 | 1640 | ($DMSO-d_6$)<br>10,5 ppm,m,1H échangeable;<br>7,9 ppm,d,1H; 7 à 7,7 ppm,m,7H;<br>3,8 ppm,AB,2H; 3,4 ppm,m,2H;<br>3,1 ppm,s,3H; 3 ppm,m,6H;<br>1,3 ppm,t,3H; 1,2 ppm,t,3H |
| 13 | 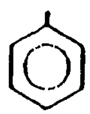 | 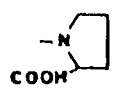 | - Cl | 1815 | 1640 (amide)<br>1720 (acide) | ($CD_3OD$)<br>7,1 à 7,9 ppm,m,8H; 4,3 ppm,m,1H;<br>3,7 ppm,s,2H; 3,6 ppm,m,2H;<br>2,1 ppm,m,4H |
| 14 | 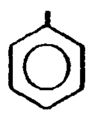 | 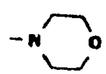 | - Cl | 1810 | 1640 | ($CDCl_3$ + $DMSO-d_6$)<br>6,5 à 7,6 ppm,m,8H; 3,5 ppm,m,10H |
| 15 | 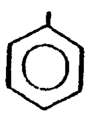 | 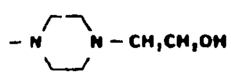 | - Cl | 1810 | 1645 | ($DMSO-d_6$)<br>7,7 ppm,d,1H; 7,3 à 7,6 ppm,m,7H;<br>2,8 à 4,5 ppm,m,14H + 1H échangeable |

**TABLEAU I**
**(Suite 4)**

| Ex | $R_1$ | R | $R_2$ | IR ($cm^{-1}$) ν C = 0 lactone | IR ($cm^{-1}$) ν C = 0 extracyclique | RMN (solvant) |
|---|---|---|---|---|---|---|
| 16 | 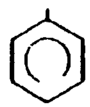 | 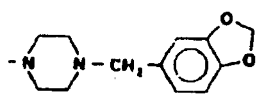 | - Cl | 1815 | 1640 | ($DMSO-d_6$)<br>11,8 ppm,m,1H échangeable;<br>7,7 ppm,d,1H; 6,7 à 7,5 ppm,m,10H;<br>6 ppm,s,2H; 2,6 à 4,5 ppm,m,12H |
| 17 | 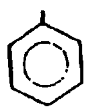 | 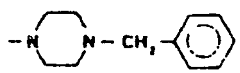 | - Cl | 1795 | 1645 | ($CDCl_3$)<br>6,7 à 7,6 ppm,m,13H; 3,1 à 3,7 ppm,m,8H;<br>2,1 à 2,6 ppm,m,4H |
| 18 | 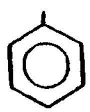 | 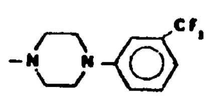 | - Cl | 1795 | 1645 | ($DMSO-d_6$)<br>6,8 à 7,9 ppm,m,12H;<br>5,6 ppm,s,1H échangeable;<br>3,9 ppm,AB,2H; 2,8 à 3,9 ppm,m,8H |
| 19 | 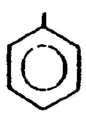 | 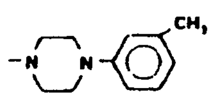 | - Cl | 1815 | 1650 | ($DMSO-d_6$)<br>5,6 à 7,8 ppm,m,12H + 1H échangeable<br>3 à 4,2 ppm,m,10H |

**TABLEAU I**
**(Suite 5)**

| Ex | $R_1$ | R | $R_2$ | IR ($cm^{-1}$) $\nu C=0$ lactone | IR ($cm^{-1}$) $\nu C=0$ extracyclique | RMN (solvant) |
|---|---|---|---|---|---|---|
| 20 | 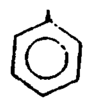 | 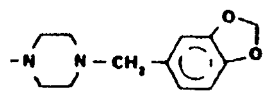 | - F | 1800 | 1640 | ($CDCl_3$ + DMSO-$d_6$)<br>12,7 ppm,m,1H échangeable;<br>6,5 à 7,8 ppm,m,11H; 6 ppm,s,2H;<br>2,8 à 4,5 ppm,m,12H |
| 21 | 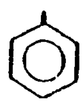 | 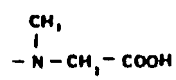 | - H | 1810 | 1655 (amide)<br>1725 (acide) | (DMSO-$d_6$)<br>7 à 7,7 ppm,m,9H + 1H échangeable;<br>3,3 à 4 ppm,m,4H;<br>3,1 ppm,s, et 2,7 ppm,s,3H |
| 22 | 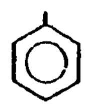 | 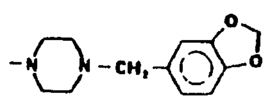 | - H | 1795 | 1650 | ($CDCl_3$)<br>6,5 à 7,7 ppm,m,12H; 5,9 ppm,s,2H;<br>3,2 à 3,6 ppm,m,8H; 2,1 à 2,6 ppm,m,4H |
| 23 | - H | 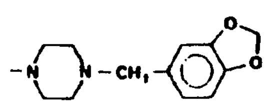 | - H | 1805 | 1645 | ($CDCl_3$)<br>6,3 à 7,7 ppm,m,7H; 5,9 ppm,s,2H; 4,1 ppm, d d,1H; 2,6 à 3,8 ppm,m,8H;<br>2,1 à 2,6 ppm,m,4H |

TABLEAU I
(Suite 6)

| Ex | $R_1$ | R | $R_2$ | IR ($cm^{-1}$) $\nu C=0$ lactone | IR ($cm^{-1}$) $\nu C=0$ extracyclique | RMN (solvant) |
|---|---|---|---|---|---|---|
| 24 | -H | 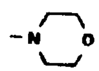 | -H | 1810 | 1640 | ($CDCl_3$)<br>6,7 à 7,5 ppm,m,4H; 4,1 ppm,d d,1H;<br>3,2 à 3,8 ppm,m,8H; 3 ppm,m,2H |
| 25 | -H | 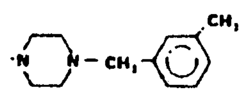 | -H | 1810 | 1650 | ($CDCl_3$)<br>6,3 à 7,7 ppm,m,8H; 4,1 ppm,d d,1H,<br>2,7 à 3,9 ppm,m,10H; 2,3 ppm,s,3H |
| 26 | -H | 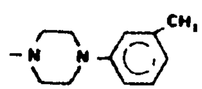 | -Cl | 1810 | 1650 | ($CDCl_3$ + $CF_3CO_2H$)<br>6,7 à 7,7 ppm,m,7H; 4,2 ppm,m,5H;<br>3,7 ppm,m,4H; 3,3 ppm,d,2H;<br>2,4 ppm,s,3H |
| 27 | 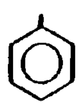 | 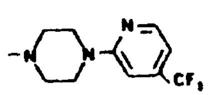 | -Cl | 1810 | 1640 | (DMSO-$d_6$)<br>8,5 ppm,d,1H; 6,85 à 7,7 ppm,m,10H;<br>3,3 à 4 ppm,m,10H |

**TABLEAU I**
**(Suite 7)**

| Ex | $R_1$ | R | $R_2$ | IR ($cm^{-1}$) |  | RMN (solvant) |
|---|---|---|---|---|---|---|
|  |  |  |  | ν C = 0 lactone | ν C = 0 extracyclique |  |
| 28 | 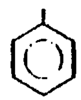 | 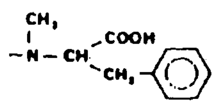 | - Cl | 1800 | 1640 (amide)<br>1720 (acide) | ($CDCl_3$)<br>6,9 à 7,5 ppm,m,13H;<br>5,25 ppm,m,1H échangeable<br>2,7 à 3,4 ppm,m,5H;2,8 ppm,s,3H |
| 29 | 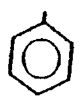 | 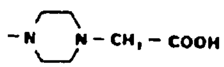 | - Cl | 1815 | 1645 (amide)<br>1750 (acide) | (DMSO-$d_6$)<br>6,8 à 7,8 ppm,m,8H; 6,5 à 4,5 ppm ,m,<br>2H échangeables; 3 à 4,3 ppm,m,12H |
| 30 | 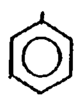 | 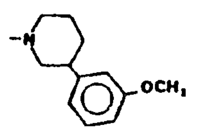 | - Cl | 1795 | 1635 | ($CDCl_3$ + DMSO-$d_6$)<br>7,2 à 8 ppm,m,8H; 6,6 à 7 ppm,m,4H;<br>3,5 à 4,2 ppm,m,4H;3,75 ppm, s,3H;<br>3,7 ppm,s,2H; 2,6 à 3,4 ppm, m,1H;<br>1,4 à 2,1 ppm,m,4H |
| 31 | 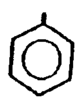 | 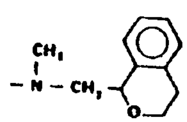 | - Cl | 1800 | 1655 | ($CDCl_3$)<br>7 à 7,5 ppm,m,12H; 4,7 à 5,1 ppm,m,1H;<br>3,5 à 4,5 ppm,m,2H; 3,3 à 3,8 ppm,m,2H;<br>3,1 ppm,s,3H; 2,6 à 2,9 ppm,m,4H |

TABLEAU I

(Suite 8)

| Ex | $R_1$ | R | $R_2$ | IR ($cm^{-1}$) $\nu C=0$ lactone | IR ($cm^{-1}$) $\nu C=0$ extracyclique | RMN (solvant) |
|---|---|---|---|---|---|---|
| 32 | 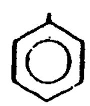 | 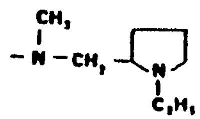 | -Cl | 1810 | 1645 | (DMSO-$d_6$) 9,5 à 10,3 ppm,m,1H échangeable 7 à 7,8 ppm,m,8H; 2,8 à 4,3 ppm,m,12H; 1,5 à 2,3 ppm,m,4H; 1 à 1,5 ppm,m,3H |
| 33 | 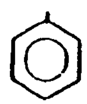 | 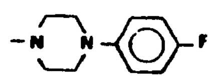 | $-CH_3$ | 1800 | 1655 | ($CDCl_3$) 6,7 à 7,7 ppm,m,12H; 3,5 à 3,8 ppm,m,4H; 3,55 ppm,s,2H; 2,8 à 3,2 ppm,m,4H; 2,4 ppm,s,3H |
| 34 | $-CH_2-CH_2-CH_3$ | 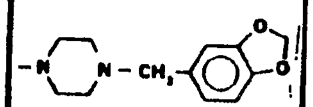 | -H | 1800 | 1640 | (DMSO-$d_6$) 7 à 7,6 ppm,m,1H échangeable; 6,8 à 7,5 ppm,m,7H; 6,1 ppm,s,2H; 2,8 à 4,4 ppm,m,12H; 1,5 à 2 ppm,m,2H; 1 à 1,6 ppm,m,2H; 0,6 à 0,9ppm,m,3H |

**TABLEAU I**
**(Suite 9)**

| Ex | $R_1$ | R | $R_2$ | IR ($cm^{-1}$) v c = 0 lactone | IR ($cm^{-1}$) v c = 0 extracyclique | RMN (solvant) |
|---|---|---|---|---|---|---|
| 35 | 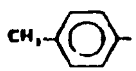 | 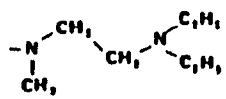 | - Cl | 1805 | 1645 | ($CDCl_3$)<br>7,5 ppm,d,1H; 6,8 à 7,5 ppm,m,6H;<br>3,4 à 4,4 ppm,m,4H; 3,2 ppm,s,3H;<br>2,7 à 3,4 ppm,m,6H; 2,3 ppm,s,3H;<br>1,3 ppm,t,6H |
| 36 | 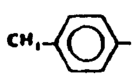 | 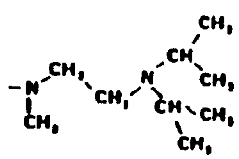 | - Cl | 1800 | 1645 | ($CDCl_3$)<br>10 à 10,5 ppm,m,1H échangeable;<br>7 à 7,6 ppm,m,7H; 3,3 à 4,1 ppm,m,4H;<br>3,2 ppm,s,3H; 2,5 à 3,2 ppm,m,4H;<br>2,3 ppm,s,3H; 1 à 2 ppm,m,12H |
| 37 | 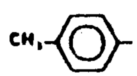 | 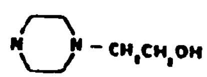 | - Cl | 1805 | 1640 | (DMSO-$d_6$)<br>7,2 à 7,7 ppm,m,7H; 2,6 à 4,3 ppm,m,14H;<br>2,5 à 4 ppm,m,2H échangeables;<br>2,3 ppm,s,3H |

ETUDE PHARMACOLOGIQUE

**EXEMPLE 38**

Hypoxie aiguë chez la souris

Des souris CD1 (Charles River), de sexe mâle, ayant reçu 30 minutes auparavant par voie intrapéritonéale le composé à tester ou un composé de référence, sont soumises à une hypoxie aiguë de type hypobare. Pour cela, elles sont placées dans une enceinte où la pression atmosphérique peut être abaissée rapidement (en 30 secondes) à une valeur de 160 mbar, ce qui provoque la mort de tous les animaux, 15 secondes environ après l'obtention de cette pression hypoxique.

La survie du cerveau est appréciée par la mesure du temps d'apparition du dernier gasp respiratoire.

Le temps de survie d'un lot traité est comparé à celui d'un lot témoin ne recevant que le solvant.

Le pourcentage d'augmentation du temps de survie après traitement des animaux avec les composés de l'invention est indiqué dans le tableau II. (Valeurs soulignées $p < 0.05$).

Comme le tableau II le démontre, les composés de l'invention exercent un puissant effet antihypoxique très supérieur à celui des composés de référence. En effet, à la dose de 100 mg/kg, le méclofenoxate, le pyritinol et le piracétam n'augmentent le temps de survie cérébrale des animaux que de 22%, 27% et 11% respectivement. La protection n'est significative que pour le pyritinol.

A la même dose, les composés de l'invention ont un effet protecteur, 2 à 8 fois plus puissant que ce dernier composé. L'augmentation du temps de survie est par exemple de 100% pour le composé des exemples 16 et 20, 126% pour le composé de l'exemple 22, supérieure à 130% pour les composés des exemples 15 et 26, et supérieure à 180% pour les composés des exemples 9 et 12.

TABLEAU II

Pourcentage d'augmentation du temps de survie

| COMPOSES | DOSES mg/kg (I.P.) | | | | | | | |
|---|---|---|---|---|---|---|---|---|
| | 3 | 10 | 30 | 100 | 200 | 300 | 600 | 1000 |
| Meclofenoxate | | | + 4 | + 22 | | | + 190 | |
| Pyritinol | | | + 13 | + 27 | + 77 | | | |
| Piracetam | | | | + 11 | | | | + 25 |
| Ex. 1 | | | | + 46 | | | | |
| Ex. 2 | | | | + 38 | | | | |
| Ex. 3 | | | | + 29 | | | | |
| Ex. 4 | | | | + 17 | | | | |
| Ex. 5 | | | | + 69 | | | | |
| Ex. 6 | | | | + 41 | | | | |
| Ex. 7 | | | | + 14 | | + 156 | | |
| Ex. 8 | | | | + 52 | | | | |
| Ex. 9 | + 65 | + 82 | + 77 | + 183 | | | | |
| Ex. 10 | | | | + 97 | | | | |
| Ex. 11 | | | | + 159 | | | | |
| Ex. 12 | + 32 | + 41 | + 84 | + 185 | | | | |
| Ex. 13 | | | | + 8 | | | | |
| Ex. 14 | | | | + 88 | | | | |
| Ex. 15 | | + 34 | + 41 | + 139 | + 203 | | | |
| Ex. 16 | | | + 64 | + 100 | | | | |
| Ex. 17 | | | + 40 | + 66 | | | | |
| Ex. 18 | | + 41 | + 53 | + 88 | | + 103 | | |
| Ex. 19 | | | | + 76 | | | | |
| Ex. 20 | | + 52 | + 104 | + 100 | | + 128 | | |
| Ex. 21 | | | | + 14 | | | | |
| Ex. 22 | | + 11 | + 42 | + 126 | | | | |
| Ex. 26 | | + 52 | + 53 | + 135 | | + 190 | | |
| Ex. 27 | | + 36 | + 59 | + 76 | | | | |
| Ex. 28 | | | | + 90 | | | | |
| Ex. 29 | | | + 32 | + 66 | | | | |
| Ex. 30 | | + 32 | + 36 | + 103 | | | | |
| Ex. 32 | | | + 26 | | | | | |

**EXEMPLE 39**

Hypoxie aiguë chez le rat

Des rats Fischer 344 (Charles River), de sexe mâle, ayant reçu 30 minutes auparavant le composé à tester ou un composé de référence, sont soumis à un défaut d'apport en oxygène en étant placés dans une enceinte normobare dans laquelle la composition du mélange gazeux circulant peut être changée avec précision. Alors que les rats témoins respirent un mélange gazeux contenant 21% d'oxygène et 79% d'azote, les rats hypoxiés respirent durant 2 minutes un mélange de 3% d'oxygène et 97% d'azote.

A la fin de la période hypoxique, les animaux sont rapidement sacrifiés par immersion totale dans l'azote liquide. Le cerveau congelé est prélevé et les composés énergétiques (ATP, ADP, AMP) sont extraits et dosés par la méthode de luminescence de la luciférine.

La charge énergétique tissulaire (EC) est calculée selon la formule d'ATKINSON :

$$EC = \frac{ATP + \frac{1}{2} ADP}{ATP + ADP + AMP}$$

Les résultats de cette étude sont indiqués dans le tableau III.

L'hypoxie entraîne chez les animaux témoins, un effondrement du taux d'ATP tissulaire (–74,2%) qui s'accompagne d'une hausse des composés mono- et di-phosphatés (AMP, ADP). C'est ce que traduit la baisse de la charge énergétique totale (–25,3%).

A la dose de 300 mg/kg, le piracétam n'exerce que très peu de protection en inhibant de 3,9% seulement les effets de l'hypoxie sur le taux d'ATP.

A la même dose, le méclofenoxate inhibe de 59,7% et de 69,6% les effets de l'hypoxie respectivement sur le taux d'ATP et la charge énergétique. Un tel effet est observé avec une dose de 100 mg/kg ou 30 mg/kg de composés de l'invention.

Dans les mêmes conditions, le pyritinol à la dose de 100 mg/kg n'exerce qu'un effet plus modeste. La chute d'ATP est inhibée de 23% tandis que celle de la charge énergétique est inhibée de 35%. Il est nécessaire d'administrer la forte dose de 300 mg/kg pour observer un net effet antihypoxique.

TABLEAU III

HYPOXIE AIGUE CHEZ LE RAT

| | | |
|---|---|---|
| Rats témoins en normoxie | : ATP = 2,373 µmoles/g | EC = 0,959 |
| Rats témoins en hypoxie | : ATP = - 74,2 % | EC = - 25,3% |

| COMPOSES | DOSE mg/kg I.P. | % D'INHIBITION DES EFFETS DE L'HYPOXIE | |
|---|---|---|---|
| | | ATP | EC |
| Méclofenoxate | 300 | 59,7 | 69,6 |
| Pyritinol | 030<br>100<br>300 | 11,1<br>23,3<br>90,4 | 16,6<br>35,2<br>85,4 |
| Piracétam | 300 | 3,9 | 1,6 |
| Exemple 1 | 100 | 54 | 61,7 |
| Exemple 9 | 030 | 65,6 | 60,1 |
| Exemple 16 | 100 | 77,5 | 76,3 |
| Exemple 20 | 030<br>100 | 56,9<br>70,2 | 61,3<br>71,5 |
| Exemple 25 | 100 | 50,5 | 52,6 |

PREPARATION PHARMACEUTIQUE

**EXEMPLE 40**

Gélules dosées à 20 mg de chlorhydrate du (dihydro-2,3 fluoro-5 oxo-2 phényl-3 benzofurannyl-3)-2 [(méthylène dioxy-3,4 benzyl)-4 pipérazinyl]-1 oxo-1 éthane

| | |
|---|---|
| Chlorhydrate du (dihydro-2,3 fluoro-5 oxo-2 phényl-3 benzofurannyl-3)-2 [(méthylène dioxy-3,4 benzyl)-4 pipérazinyl]-1 oxo-1 éthane | 20 mg |
| Amidon de maïs | 15 mg |
| Lactose | 25 mg |
| Talc | 5 mg |

Pour une gélule N° 3.

## Revendications

1. Composés de formule générale I,

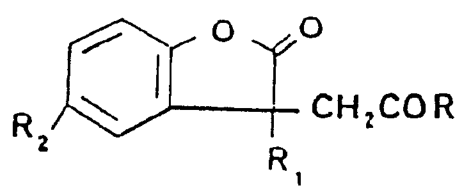

(I)

dans laquelle :

– $R_1$ représente un atome d'hydrogène, un radical alkyle linéaire ou ramifié, de 1 à 4 atomes de carbone ou un radical phényle éventuellement substitué par un atome d'halogène, par radical alcoxy renfermant de 1 à 4 atomes de carbone ou un radical alkyle de 1 à 4 atomes de carbone,

– $R_2$ représente un atome d'hydrogène, ou d'halogène, un radical hydroxy, un radical alkyle de 1 à 4 atomes de carbone ou un radical alcoxy renfermant de 1 à 4 atomes de carbone,

– R représente

ou

un radical hydroxyle, un radical alcoxy linéaire ou ramifié de 1 à 4 atomes de carbone, ou un radical benzyloxy,

ou

un radical de formule générale A

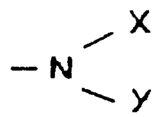

(A)

dans laquelle,

– X et Y identiques ou différents représentent chacun

- un radical alkyle linéaire ou ramifié renfermant de 1 à 5 atomes de carbone,
- un radical de formule générale $A_1$

$$-CHZ-COOH \qquad (A_1)$$

dans laquelle Z représente un atome d'hydrogène, un radical alkyle linéaire ou ramifié de 1 à 4 atomes de carbone, un radical hydroxyalkyle de 1 à 4 atomes de carbone, un radical imidazolyle-4 méthylène, ou un radical benzyl éventuellement substitué par un radical alkyle de 1 à 4 atomes de carbone ou par un radical hydroxyle,

- un radical de formule générale $A_2$

$$-CH_2W \qquad (A_2)$$

dans laquelle W représente un radical dialkylaminométhylène linéaire ou ramifié de 3 à 9 atomes de carbone, un radical pyrrolidinyle-2 éventuellement substitué par un alkyle de 1 à 4 atomes de carbone, un radical benzyle, un radical isochromannyle-1 ou un radical isoquinolyle-1,

- ou forment ensemble avec l'azote auquel ils sont attachés un radical carboxy-2 pyrrolidinyle-2, un radical morpholinyle-4, un radical pipéridino éventuellement substitué par un radical phényle ou alcoxyphényle de 7 à 10 atomes de carbone, ou un radical pipérazinyle-1 (éventuellement substitué en 4 par un radical hydroxyalkyle de 1 à 4 atomes de carbone, un radical carboxyalkyle de 2 à 5 atomes de carbone, un radical benzyle éventuellement substitué par un radical alkyle de 1 à 4 atomes de carbone, un radical méthylène dioxy-3,4 benzyle, un radical phényle éventuellement substitué par un radical alkyle de 1 à 4 atomes de carbone ou par un radical trifluorométhyle ou par un atome d'halogène, ou un radical pyridyle-2 éventuellement substitué par un radical alkyle de 1 à 4 atomes de carbone ou par un radical trifluorométhyle),

sous forme racémique ou d'isomères optiques,

et leurs sels d'addition à un acide minéral ou organique pharmaceutiquement acceptable, lorsqu'ils contiennent un groupement salifiable basique ou leurs sels d'addition à une base minérale ou organique pharmaceutique acceptable, lorsqu'ils contiennent un groupement salifiable acide.

2. Le (fluoro-5 dihydro-2,3 oxo-2 phényl-3 benzofurannyl-3)-2 [(méthylène dioxy-3,4 benzyl)-4 pipérazinyl]-1 oxo-1 éthane et ses sels d'addition à un acide pharmaceutiquement acceptable.

3. La N-[(chloro-5 dihydro-2,3 oxo-2 phényl-3 benzofurannyl-3)-2 acétyl], N-méthyl phénylalanine et ses sels d'addition à une base pharmaceutiquement acceptable.

4. Le (chloro-5 dihydro-2,3 oxo-2 phényl-3 benzofurannyl-3)-2 [(trifluorométhyl-5 pyridinyl-2)-4 pipérazinyl]-1 oxo-1 éthane et ses sels d'addition à un acide pharmaceutiquement acceptable.

5. Le N-méthyl N-diéthylaminoéthyl (chloro-5 dihydro-2,3 oxo-2 phényl-3 benzofurannyl-3)-2 acétamide et ses sels d'addition à un acide pharmaceutiquement acceptable.

6. Procédé de préparation des composés de formule générale I, caractérisé en ce que :

l'on condense une benzofurannone-2 de formule générale II

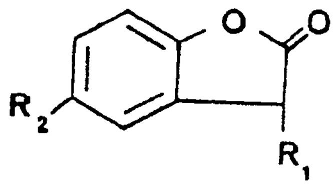

(II)

dans laquelle $R_1$ et $R_2$ ont la signification précédemment définie pour la formule I, avec un bromoacétate d'alkyle ou de benzyle de formule générale III,

$$BrCH_2COR'$$

(III)

dans laquelle $R^1$ représente un radical alcoxy de 1 à 4 atomes de carbone ou un radical benzyloxy, pour former un dérivé de formule générale Ia

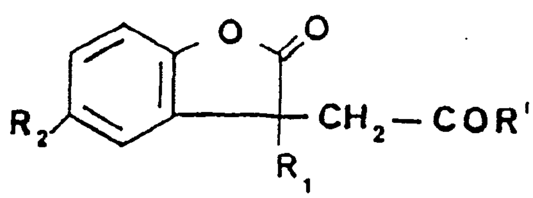

(Ia)

dans laquelle la définition des substituants $R^1$, $R_1$ et $R_2$ demeure cellementionnée ci-dessus,
lequel ensuite,
que l'on peut soumettre à une hydrogénation catalytique pour obtenir un dérivé de l'acide (dihydro-2,3 oxo-2 benzofurannyl-3)-2 acétique de formule générale Ib,

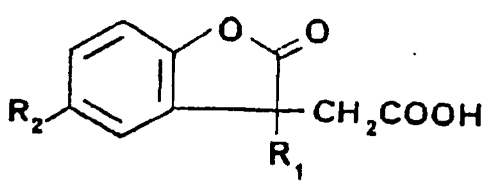

(Ib)

dans laquelle $R_1$, et $R_2$ ont la signification précédemment définie,
lequel ensuite
– ou
que l'on salifie, si l'on désire, avec une base pharmaceutiquement acceptable,
• ou
pour former les amides correspondants,
que l'on soumet d'abord à l'action du chlorure de thionyle pour obtenir un halogénure d'acyle de formule générale IV :

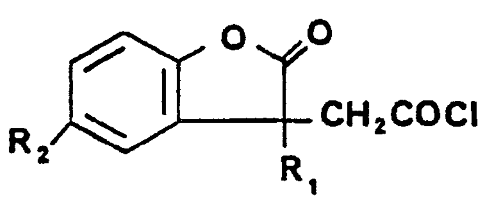

(IV)

dans laquelle la définition des substituants $R_1$ et $R_2$ demeure celle mentionnée pour la formule I selon la revendication 1,

et que l'on condense ensuite avec une amine secondaire de formule générale V

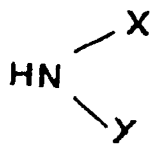

(V)

dans laquelle X et Y ont la signification précédemment définie pour la formule I pour former les composés de formule générale Ic :

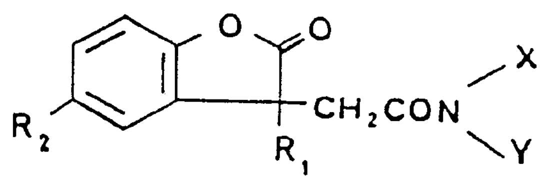

(Ic)

dans laquelle la signification des substituants $R_1$, $R_2$, X et Y reste identique à celle donnée précédemment, que l'on peut salifier ensuite, si l'on désire, avec une base pharmaceutiquement acceptable lorsqu'ils contiennent un groupement acide, ou avec un acide pharmaceutiquement acceptable lorsqu'ils contiennent un groupement basique salifiable.

7. Utilisation des composés de formule générale I, selon la revendication 1, pour la préparation d'une composition pharmaceutique destinée au traitement des maladies liées à l'hypoxémie et à l'insuffisance énergétique ou au vieillissement cérébral.

8. Composition pharmaceutique contenant comme principe actif un composé selon l'une quelconque des revendications 1 à 6, en association ou en mélange avec un excipient ou un véhicule inerte non toxique, pharmaceutiquement acceptable.

9. Composition pharmaceutique selon la revendication 8 renfermant comme principe actif au moins un composé selon les revendications 1 à 6 utilisable dans le traitement des maladies liées à l'hypoxémie et à l'insuffisance énergétique ou le vieillissement cérébral.

## Ansprüche

1. Verbindungen der allgemeinen Formel I

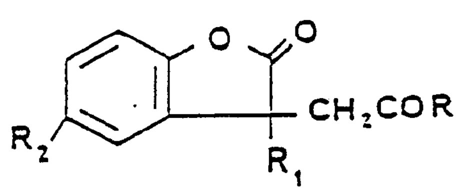

(I)

in der :

- $R_1$ ein Wasserstoffatom, eine geradkettige oder verneigte Alkylgruppe mit 1 bis 4 Kohlenstoffatomen oder eine Phenylgruppe, die gegebenenfalls durch ein Halogenatom, eine Alkoxygruppe mit 1 bis 4 Kohlenstoffatomen oder eine Alkylgruppe mit 1 bis 4 Kohlenstoffatomen substituiert ist,
- $R_2$ ein Wasserstoffatom, ein Halogenatom, eine Hydroxylgruppe, eine Alkoxygruppe mit 1 bis 4 Kohlenstoffatomen oder eine Alkoxygruppe mit 1 bis 4 Kohlenstoffatomen,
- R entweder eine Hydroxylgruppe, eine geradkettige oder verneigte Alkoxygruppe mit 1 bis 4 Kohlenstoffatomen oder eine Benzyloxygruppe, oder eine Gruppe der allgemeinen Formel A

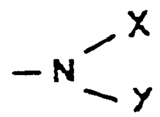

(A)

in der

– X und Y, die gleichartig oder verschieden sein können, jeweils

- geradkettige oder verzweigte Alkylgruppen mit 1 bis 5 Kohlenstoffatomen,
- Gruppen der allgemeinen Formel $A_1$

$$-CHZ-COOH \qquad (A_1)$$

in der Z ein Wasserstoffatom, eine geradkettige oder verzweigte Alkylgruppe mit 1 bis 4 Kohlenstoffatomen, eine Hydroxyalkylgruppe mit 1 bis 4 Kohlenstoffatomen, eine Imidazol-4-yl-methylengruppe oder eine Benzylgruppe, die gegebenenfalls durch eine Alkylgruppe mit 1 bis 4 Kohlenstoffatomen oder eine Hydroxylgruppe substituiert ist,

- Gruppen der allgemeinen Formeln $A_2$

$$-CH_2W \qquad (A_2)$$

in der W eine geradkettige oder verzweigte Dialkylaminomethylengruppe mit 3 bis 9 Kohlenstoffatomen, eine Pyrrolidin-2-yl-gruppe, die gegebenenfalls durch eine Alkylgruppe mit 1 bis 4 Kohlenstoffatomen substituiert ist, eine Benzylgruppe, eine Isochroman-1-yl-gruppe oder eine Isochinol-1-yl-gruppe darstellt,

- oder gemeinsam mit dem Stickstoffatom, das sie gebunden sind, eine 2-Carboxy-pyrrolidin-2-yl-gruppe, eine Morpholin-4-yl-gruppe, eine Piperidinogruppe, die gegebenenfalls durch eine Phenylgruppe oder eine Alkoxyphenylgruppe mit 7 bis 10 Kohlenstoffatomen substituiert ist, oder eine Piperazin-1 -yl-gruppe (die gegebenenfalls in der 4-Stellung durch eine Hydroxyakylgruppe mit 1 bis 4 Kohlenstoffatomen, eine Carboxyalkylgruppe mit 2 bis 5 Kohlenstoffatomen, eine Benzylgruppe, die gegebenenfalls durch eine Alkylgruppe mit 1 bis 4 Kohlenstoffatomen substituiert ist, eine 3,4-Dioxyethylen-bezylgruppe, eine Phenylgruppe, die gegebenefalls durch eine Alkylgruppe mit 1 bis 4 Kohlenstoffatomen oder eine Trifluormethylgruppe oder ein Halogenatom substituiert ist, oder eine Pyrid-2-yl-gruppe, die gegebenenfalls durch eine Alkylgruppe mit 1 bis 4 Kohlenstoffatomen oder eine Trifluormethylgruppe substituiert ist), darstellen, bedeuten,

in Form des Racemats oder der optischen Isomeren,

und deren Additionssalze mit einer anorganischen oder organischen, pharmazeutisch annehmbaren Säure, wenn sie eine basische, in das Salz überführbare Gruppe aufweisen, oder deren Additionssalze mit einer anorganischen oder organischen, pharmazeutisch annehmbaren Base, wenn sie eine saure, in ein Salz überführbare Gruppe enthalten.

2. 2-(5-Fluor-2,3-dihydro-2-oxo-3-phenyl-benzofuran-3-yl)-1-[4-(3,4-methylendioxy-benzyl)-piperazinyl-1-oxo-ethan als Verbindung der allgemeinen Formel (I) gemaß Anspruch 1 und dessen Additionssalze mit einer pharmazeutisch annehmbaren Säure.

3. [2-(5-Chlor-2,3-dihydro-2-oxo-3-phenyl-benzofuran-3-yl)-acetyl]-N-methyl-phenylalanin als Verbindung der allgemeinen Formel (I) gemapß Anspruch 1 und dessen Additionssalze mit einer pharmazeutisch annehmbaren Base.

4. 2-(5-Chlor-2,3-dihydro-2-oxo-3-phenyl-benzofuran-3-yl)-1-[4-(5-trifluormethyl-pyridin-2-yl)-piperazinyl]-1-oxo-ethan als Verbindung der allgemeinen Formel (I) gemaß Anspruch 1 und dessen Additionssalze mit einer pharmazeutisch annehmbaren Säure.

5. N-Methyl-N-diethylaminoethyl-2-(5-chlor-2,3-dihydro-2-oxo-3-phenyl-benzofuran-3-yl)-acetamid als Verbindung der allgemeinen Formel (I) gemaß Anspruch 1 und dessen Additionssalze mit einer pharmazeutisch annehmbaren Säure.

6. Verfahren zur Herstellung der Verbindungen der allgemeinen Formel I gemaß Anspruch 1, **dadurch**

**gekennzeichnet**, daß man ein Benzofuran-2-on der allgemeinen Formel II

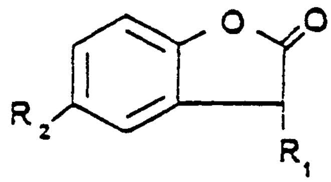

(II)

in der $R_1$ und $R_2$ die bezüglich der allgemeinen Formel I in Anspruch 1 angegebenen Bedeutungen besitzen, mit einem Alkylbromacetat oder Benzylbromacetat der allgemeinen Formel III

$BrCH_2COR^1$ (III)

in der $R^1$ eine Alkoxygruppe mit 1 bis 4 Kohlenstoffatomen oder eine Benzylyxogruppe bedeutet, zur Bildung eines Derivats der allgemeinen Formel Ia kondensiert

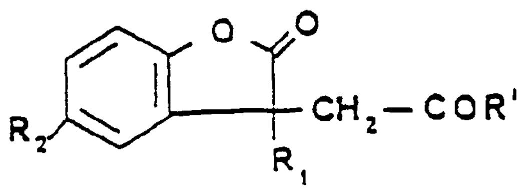

(Ia)

in der die Substituenten $R^1$, $R_1$ und $R_2$ die oben angegebenen Bedeutungen besitzen, welches man anschließend

entweder einer katalytischen Hydrierung unterwirft zur Bildung eines Derivats der 2-(2,3-Dihydro-2-oxo-benzofuran-3-yl)-essigsäure der allgemeinen Formel Ib

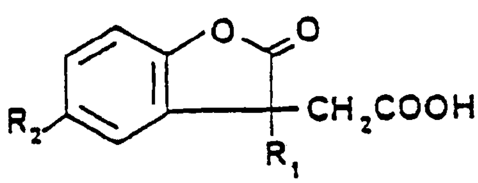

(Ib)

in der $R_1$ und $R_2$ die bezüglich der allgemeinen Formel (I) in Anspruch 1 angegebenen Bedeutungen besitzen, welche man gewünschtenfalls mit einer pharmazeutisch annehmbaren Base in ein Salz überführt oder

welche man zur Bildung der entsprechenden Amide zunächst der Einwirkung von Thionylchlorid zur Bildung eines Acylhalogenids der allgemeinen Formel IV

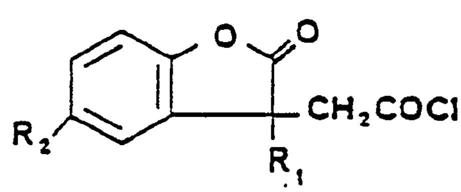

(IV)

in der die Substituenten $R_1$ und $R_2$ die bezüglich der allgemeinen Formel (I) in Anspruch 1 angegebenen Bedeutungen besitzen, unterwirft, welches man anschließend mit einem sekundären Amin der allgemeinen Formel V

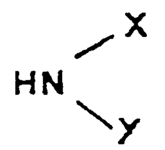

(V)

in der X und Y die oben bezüglich der allgemeinen Formel I angegebenen Bedeutungen besitzen, kondensiert zur Bildung der Verbindungen der allgemeinen Formel Ic

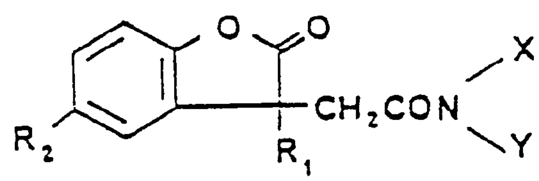

(Ic)

in der die Substituenten $R_1$ und $R_2$, X und Y die oben angegebenen Bedeutungen besitzen,
welche man anschließend gewünschtenfalls mit einer pharmazeutish annehmbaren Base, wenn sie eine saure Gruppe enthalten, oder mit einer pharmazeutisch annehmbaren Säure, wenn sie eine basische in ein Salz überführbare Gruppe enthalten, in ein Salz umwandelt.

7. Verwendung der Verbindungen der allgemeinen Formel I nach Anspruch 1 zur Herstellung einer pharmazeutischen Zubereitung zur Behandlung von Erkrankungen, die durch Hypoxämie und Energiemangel verursacht sind und der Alterung des Gehirns.

8. Pharmazeutische Zubereitung enthaltend als Wirkstoff eine Verbindung nach einem der Ansprüche 1 bis 5 in Kombination oder in Mischung mit einem inerten, nicht toxischen, pharmazeutisch annehmbaren Bindemittel oder Trägermatenal.

9. Pharmazeutische Zubereitung nach Anspruch 7 enthaltend als Wikstoff mindestens eine Verbindung nach einem der Ansprüche 1 bis 5 zur Verwendung bei der Behandlung von Erkrankungen, die durch Hypoxämie und Energiemangel verursacht sind, sowie der Alterung des Gehirns.

**Claims**

1. Compounds of the general formula I

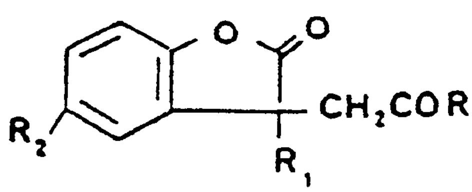

(I)

in which :

– $R_1$ represents a hydrogen atom, a linear or branched alkyl radical having from 1 to 4 carbon atoms or a phenyl radical optionally substituted by a halogen atom, by an alkoxy radical having from 1 to 4 carbon atoms or by an alkyl radical having from 1 to 4 carbon atoms,

– $R_2$ represents a hydrogen atom, a halogen atom, a hydroxy radical, an alkyl radical having from 1 to 4 carbon atoms or an alkoxy radical having from 1 to 4 carbon atoms,

– R represents

either

a hydroxy radical, a linear or branched alkoxy radical having from 1 to 4 carbon atoms, or a benzyloxy radical,

or

a radical of the general formula A

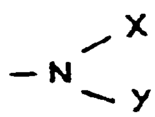

(A)

in which

– X and Y may be the same or different and each represents

- a linear or branched alkyl radical having from 1 to 5 carbon atoms,
- a radical of the general formula $A_1$

$$-CHZ-COOH \qquad (A_1)$$

in which Z represents a hydrogen atom, a linear or branched alkyl radical having from 1 to 4 carbon atoms, a hydroxyalkyl radical having from 1 to 4 carbon atoms, a 4-imidazolylmethylene radical, or a benzyl radical optionally substituted by an alkyl radical having from 1 to 4 carbon atoms or by a hydroxy radical,

- a radical of the general formula $A_2$

$$-CH_2W \qquad (A_2)$$

in which W represents a linear or branched dialkylaminomethylene radical having from 3 to 9 carbon atoms, a 2-pyrrolidinyl radical optionally substituted by an alkyl radical having from 1 to 4 carbon atoms, a benzyl radical, a 1-isochromanyl radical or a 1-isoquinolyl radical,

- or, together with the nitrogen atom to which they are bonded, form a 2-carboxy-2-pyrrolidinyl radical, a 4-morpholinyl radical, a piperidino radical optionally substituted by a phenyl or alkoxyphenyl radical having from 7 to 10 carbon atoms, or a 1-piperazinyl radical (optionally substituted at the 4-position by a hydroxy-alkyl radical having from 1 to 4 carbon atoms, a carboxyalkyl radical having from 2 to 5 carbon atoms, a benzyl radical optionally substituted by an alkyl radical having from 1 to 4 carbon atoms, a 3,4-methylenedioxybenzyl radical, a phenyl radical optionally substituted by an alkyl radical having from 1 to 4 carbon atoms or by a trifluoromethyl radical or by a halogen atom, or a 2-pyridyl radical optionally substituted by an alkyl radical having from 1 to 4 carbon atoms or by a trifluoromethyl radical),

in racemic form or in the form of optical isomers,

and, if they contain a basic salt-forming group, their addition salts with a pharmaceutically acceptable mineral or organic acid or, if they contain an acidic salt-forming group, their addition salts with a pharmaceutically acceptable mineral or organic base.

2. 2-(5-fluoro-2,3-dihydro-2-oxo-3-phenyl-3-benzofuranyl)-1-[4-(3,4-methylenedioxybenzyl)-piperazinyl]-1-oxoethane, said compound corresponding to formula I according to claim 1, and its addition salts with a pharmaceutically acceptable acid.

3. N-[2-(5-chloro-2,3-dihydro-2-oxo-3-phenyl-3-benzofuranyl)-acetyl]-N-methylphenylalanine, said compound corresponding to formula I according to claim 1, and its addition salts with a pharmaceutically acceptable base.

4. 2-(5-chloro-2,3-dihydro-2-oxo-3-phenyl-3-benzofuranyl)-1-[4-(5-trifluoromethyl-2-pyridinyl)-piperazinyl)-1-oxoethane, said compound corresponding to formula 1 according to claim 1, and its addition salts with a pharmaceutically acceptable acid.

5. N-methyl-N-diethylaminoethyl-2-(5-chloro-2,3-dihydro-2-oxo-3-phenyl-3-benzofuranyl)-acetamide, said compound corresponding to formula I according to claim 1, and its addition salts with a pharmaceutically acceptable acid.

6. Process for the preparation of compounds of the general formula I according to claim 1, characterised in that a 2-benzofuranone of the general formula II

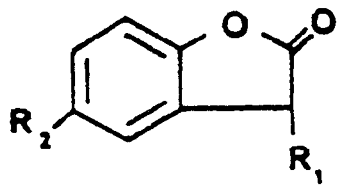

(II)

in which $R_1$ and $R_2$ are as defined above for formula I according to claim 1, is condensed with an alkyl orbenzyl bromoacetate of the general formula III

$BrCH_2COR'$ (III)

in which R′ represents an alkoxy radical having from 1 to 4 carbon atoms or a benzyloxy radical,
to form a derivative of the general formula Ia

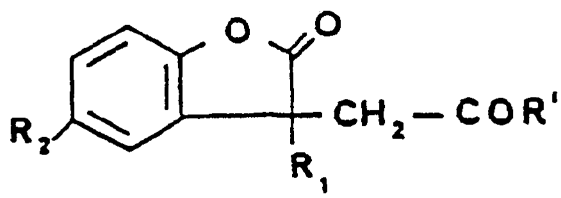

(Ia)

in which the definitions of the substituents R′, $R_1$ and $R_2$ are as specified above,
which may then be subjected to catalytic hydrogenation to obtain a derivative of 2-(2,3-dihydro-2-oxo-3-benzofuranyl)-acetic acid of the general formula Ib

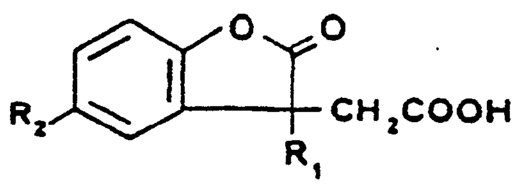

(Ib)

in which $R_1$ and $R_2$ are as defined above for formula I according to claim 1,
which is then
• either,
if desired formed into a salt with a pharmaceutically acceptable base
• or,
in order to form the corresponding amides is subjected first to the action of thionyl chloride to obtain an acyl halide of the general formula IV :

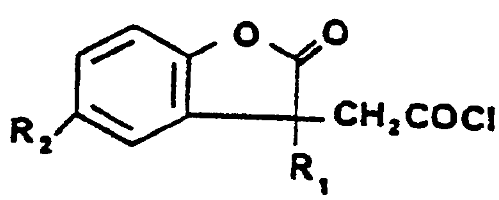

(IV)

in which the definition of the substituents $R_1$ and $R_2$ is as specified for formula I according to claim 1,
and this is then condensed with a secondary amine of the general formula V

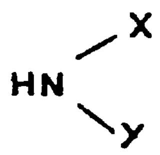

(V)

in which X and Y are as defined hereinbefore for formula I, in order to form compounds of the general formula Ic :

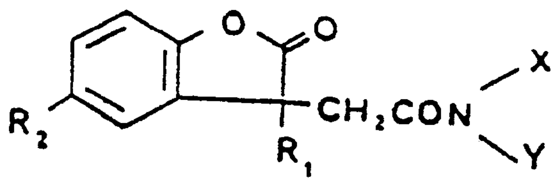

(Ic)

in which the meanings of the substituents $R_1$, $R_2$, X and Y are identical to those given hereinbefore,

which may then, if desired, if they contain an acidic group, be formed into a salt with a pharmaceutically acceptable base or, if they contain a salt-forming basic group, be formed into a salt with a pharmaceutically acceptable acid.

7. Use of the compounds of the general formula I according to claim 1 for the preparation of a pharmaceutical composition for use in the treatment of disorders associated with hypoxaemia and energy deficiency or cerebral ageing.

8. Pharmaceutical composition containing as active ingredient a compound according to any one of claims 1 to 5, in association or admixture with an excipient or a pharmaceutically acceptable inert non-toxic carrier.

9. Pharmaceutical composition according to claim 8, containing as active ingredient at least one compound according to claims 1 to 5 which can be used for the treatment of disorders associated with hypoxaemia and energy deficiency or cerebral ageing.